Europäisches Patentamt

European Patent Office

Office européen des brevets

(19)

(11) Publication number : **0 494 508 A1**

# (12) EUROPEAN PATENT APPLICATION

(21) Application number : **91311337.9**

(22) Date of filing : **05.12.91**

(51) Int. Cl.⁵ : **C07D 487/22,** B01J 31/18,
// (C07D487/22, 257:00,
209:00, 209:00, 209:00,
209:00)

(30) Priority : **07.12.90 US 634261**

(43) Date of publication of application :
**15.07.92 Bulletin 92/29**

(84) Designated Contracting States :
**BE DE FR GB IT NL**

(71) Applicant : **Sun Refining and Marketing
Company
1801 Market Street
Philadelphia, PA 19103-1699 (US)**

(72) Inventor : **Ellis, Paul E., Jr.
1179 Glenside Avenue
Downingtown, PA 19335 (US)**
Inventor : **Lyons, James E.
211 Cooper Drive
Wallingford, PA 19086 (US)**
Inventor : **Langdale, Wayne A.
928 Reese Avenue
Milmont Park, PA 19033 (US)**

(74) Representative : **Lewin, John Harvey
Elkington and Fife Prospect House 8
Pembroke Road
Sevenoaks, Kent TN13 1XR (GB)**

(54) Improved process for preparing perhaloporphyrin complexes.

(57)    The invention relates to a method of preparing perhalogenated porphyrins which comprises reacting starting material comprising a metal tetrapentafluorophenylporphyrin or tetraperfluoro($C_1$-$C_3$)alkylporphyrin, said metal being iron, manganese, chromium or ruthenium, with bromine for a time sufficient to brominate the pyrrolic hydrogens in said pyrrole, and recovering as product metal tetrakis(pentafluorophenyl)-octabromoporphyrin or tetraperfluoro($C_1$-$C_3$)alkyloctabromoporphyrin.

EP 0 494 508 A1

Jouve, 18, rue Saint-Denis, 75001 PARIS

## Background of Invention

U. S. Patent 4,895,682 discloses that perhalogenated metal ligands are excellent catalysts for the oxidation of alkanes. Preferably, the halogen is fluorine. The present invention provides a method of preparing certain such ligands, namely perhalogenated metal (tetraphenyl or tetraalkyl) porphyrins such as irontetrakispentafluorophenyloctabromoporphyrinato chloride, which is often written as $Fe(TPPF_{20}Br_8)Cl$. The use of a beta sign before the "$Br_8$", to refer to the pyrrolic positions, is sometimes dispensed with for simplicity. Also, the technically correct tetrakis is sometimes written as tetra.

Perhalogenation of tetraphenylporphyrin (TPP) requires twenty-eight halogens. It can be relatively easy to add twenty fluorines i.e., five on each of the four phenyl rings (by prior fluorination of starting materials), but the remaining eight pyrrolic hydrogens are more difficult to replace. For example, Dolphin, U. S. Patent 4,892,941, shows the perchlorination of Fe-$(TPPCl_{20})Cl$ to yield $Fe(TPPCl_{28})Cl$. However, when $Fe(TPPF_{20})Cl$ is chlorinated under the same conditions employed by Dolphin, little additional halogenation occurs.

Likewise, direct fluorination of $Fe(TPPF_{20})Cl$ is ineffective to add fluorine in the beta positions of the TPP molecule to complete the fluorination.

On the other hand, if the iron $(TPPF_{20})Cl$ is converted to the zinc salt, $Zn(TPPF_{20})Cl$, by known procedures (or the zinc salt is made in the first instance), the zinc salt can be directly fluorinated to $Zn$-$(TPPF_{28})Cl$, and this, followed by removal of the zinc and insertion of iron, yields the perfluorinated iron porphyrin, $Fe(TPPF_{28})Cl$. However the plurality of steps involved makes this scheme less attractive.

Another method known in the art to complete the halogenation of $Fe(TPPF_{20})$ salt also involves preparation of the zinc salt, reaction of the zinc salt with n-bromo-succinimide to introduce eight bromine atoms, followed by insertion of iron or other desired metal. This procedure is disclosed by Tsuchiya and Traylor in *Inorganic Chemistry, 26*, 1987, 1338. It is a multistep process which gives poor yields of impure products which are difficult to purify. Impure products obtained by this and other multi-step processes generally do not have the high catalytic activity we desire.

The same situation presents itself with tetraalkylporphyrins; the exposed alkyl hydrogens are easier to replace with halogen than the interior pyrrolic hydrogens.

## Summary of Invention

Compounds such as $Fe(TPPF_{20})Cl$ are converted to $Fe(TPPF_{20}Br_8)Cl$ by reaction with bromine. Iron(tetrakispentafluorophenyl)porphyrinato is perhalogenated by reaction with bromine in one embodiment.

## Detailed Description of Invention

The starting materials of our invention are metal (tetraperfluorophenyl)porphyrins or metal (tetraperfluoroalkyl)porphyrins. The metal is preferably iron but can also be chromium, manganese, or ruthenium.

Each porphyrin molecule contains phenyl groups or alkyl groups, with the alkyls having 1-3 carbon atoms, in the meso position. In either case it is completely fluorinated. If simply phenyl, perfluorination requires a total of 20 fluorine atoms; if alkyl the number depends upon the number of carbon atoms. Preferably the alkyl is methyl.

The metal porphyrins described above exist as a salt such as chloride, hydroxide, acetate, etc. and are properly written, e.g., as $Fe(TPPF_{20})OH$ for iron (tetrakispentafluorophenyl)hydroxide. This anion is sometimes omitted herein for simplicity since its presence is obvious and its specific identity immaterial to the chemistry involved in our invention.

The metal porphyrins described above, as well as their preparation, are known in the art and thus need not be further described. See for example, the aforesaid Dolphin and Traylor references, as well as our U. S. Patent 4,900,871 and our applications Serial No. 425,089 filed October 23, 1989, now abandoned, and Serial No. 568,116, filed August 16, 1990, all of which are incorporated herein by reference.

For convenience, our invention will be described with reference to $Fe(TPPF_{20})Cl$ as the starting material.

$Fe(TPPF_{20})Cl$ is reacted with bromine for .1-50 hours, preferably 1-20 hours at a temperature of 70-150°C, preferably 30-100°C. Since most of the startling materials are solids, carbon tetrachloride is used as a solvent. Other inert solvents can also be,used such as tetraglyme, diglyme, certain aromatic ethers, etc. The product $Fe(TPPF_{20}Br_8)Cl$ is separated from the reaction mass by chromatography on silica gel or alumina.

The product obtained by our procedure is purer than that obtained by other procedures and as a result makes a superior catalyst for the oxidation of alkanes.

## Example

150 ml bromine are added to 300 ml $CCl_4$ at room temperature under a nitrogen blanket. Next, 0.5 gm. of $Fe(TPPF_{20})Cl$ is added and the entire mixture heated to 55°C. This is less than the reflux temperature. The bromination is followed by UV analysis. Bromination begins after a short induction period and is complete after 10 hours.

The product $Fe(TPPF_{20}Br_8)Cl$ is separated by chromatography over neutral alumina. It has high activity for propane oxidation.

## Claims

1. Method of preparing perhalogenated porphyrins which comprises reacting starting material comprising a metal tetrapentafluorophenylporphyrin or tetraperfluoro($C_1$-$C_3$) alkylporphyrin, said metal being iron, manganese, chromium or ruthenium, with bromine for a time sufficient to brominate the pyrrolic hydrogens in said pyrrole, and recovering as product metal tetrakis(pentafluorophenyl)-octabromoporphyrin or tetraperfluoro($C_1$-$C_3$)alkyloctabromoporphyrin.

2. Method according to Claim 1 wherein said starting material is irontetrapentafluorophenylporphyrin and said product is irontetrapentafluorophenyloctabromoporphyrin.

3. Method according to Claim 1 wherein said reaction is at a temperature of 20-150°C for 0.1-50 hours.

4. Method according to Claim 1 wherein said reaction is carried out in the presence of a solvent for said starting material.

5. Method according to Claim 1 wherein said metal is iron.

6. Method according to Claim 1 wherein said starting material is irontetratrifluoromethylporphyrin.

7. Use of a perhalogenated porphyrin prepared by a method according to any preceding claim as a catalyst for oxidation of alkanes.

EP 0 494 508 A1

| | European Patent Office | EUROPEAN SEARCH REPORT | Application Number |
|---|---|---|---|

EP    91 31 1337

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | COORDINATION CHEMISTRY REVIEWS vol. 105, 1 November 1990, AMSTERDAM pages 181 - 193; P E ELLIS JR. ET AL: 'Selective air oxidation of light alkanes catalyzed by activated metalloporphyrins-The search for a suprabiotic system' * page 185 , paragraph 1 ; page181 , summary * | 1,7 | C07D487/22 B01J31/18 //(C07D487/22, 257:00,209:00, 209:00,209:00, 209:00) |
| | ----- | | |
| | | | **TECHNICAL FIELDS SEARCHED (Int. Cl.5 )** |
| | | | C07D B01J |

The present search report has been drawn up for all claims

| Place of search | Date of completion of the search | Examiner |
|---|---|---|
| THE HAGUE | 27 APRIL 1992 | HEYWOOD C.J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
......................................................................................
& : member of the same patent family, corresponding
    document

EPO FORM 1503 03.82 (P0401)

4